# EUROPÄISCHE PATENTSCHRIFT

(11) **EP 2 694 145 B1**
(45) Veröffentlichungstag und Bekanntmachung des Hinweises auf die Patenterteilung: **06.06.2018**
(21) Anmeldenummer: 12715047.2
(22) Anmeldetag: 05.04.2012
(51) Int. Cl.: A61M 15/00

(54) **MEDIENSPENDER**
FLUID DISPENSER
DISTRIBUTEUR DE FLUIDES

(30) Priorität: 07.04.2011 DE 102011007008
(43) Veröffentlichungstag der Anmeldung: 12.02.2014
(73) Patentinhaber: Aptar Radolfzell GmbH, 78315 Radolfzell (DE)
(72) Erfinder: HELMLINGER, Michael, 78315 Radolfzell-Böhringen (DE)
(74) Vertreter: Patentanwaltskanzlei Cartagena
(86) Internationale Anmeldenummer: PCT/EP2012/056351
(87) Internationale Veröffentlichungsnummer: WO 2012/136805

(56) Entgegenhaltungen:
- GB-A- 2 469 068
- US-A- 5 363 842
- US-A1- 2007 135 756
- US-A1- 2009 151 721
- US-B1- 6 202 642

## Beschreibung

### Anwendungsgebiet und Stand der Technik

Die Erfindung betrifft einen transportablen Mediumspender zum Austrag von flüssigen, pastösen oder pulverförmigen Medien mit einem Gehäuse mit einer Austragöffnung, mit einem Mediumspeicher zur Aufnahme des Mediums vor dem Austrag und mit einer Betätigungshandhabe, mittels derer ein Fördervorgang bewirkt werden kann, bei dem Medium vom Mediumspeicher zur Austragöffnung gefördert wird.

Gattungsgemäße Mediumspender sind aus dem Stand der Technik allgemein bekannt. Sie finden beispielsweise zur Handhabung von Lacken oder kosmetischen Flüssigkeiten Verwendung. Insbesondere betrifft die vorliegende Erfindung gattungsgemäßer Spender, die bestimmungsgemäße zum Austrag von pharmazeutischen Medien vorgesehen sind. Das in einem gattungsgemäßen Spender vorgehaltenen Medium kann mittels der Betätigungshandhabe ausgetragen werden, wobei der hierfür erforderliche Fördervorgang von der Energie der Betätigung gespeist sein kann oder auch durch eine elektrische Energie oder ein ebenfalls vorgehaltenes Treibmittel bewirkt werden kann.

Eine Vielzahl auszutragender Medien bestehen aus mehreren Komponenten, die bestimmungsgemäß in durchmischter Form ausgetragen werden sollen. Solche Medien können beispielsweise in Form von Suspensionen oder Emulsionen vorliegen. Es kann sich jedoch auch um homogene Mischungen handeln. Bei all diesen Medien, die aus mehreren Komponenten bestehen, besteht die Problematik, dass diese sich über einen längeren Zeitraum im Mediumspeicher entmischen können, so dass beim Austrag des Mediums nicht das gewünschte Mischungsverhältnis erzielt wird.

Zwar ist es bei Spendern, die zum Austrag solcher Medien vorgesehen sind, üblich, dass diese zusammen mit einer Handhabungsanweisung ausgeliefert werden, der sich der Hinweis entnehmen lässt, das der Mediumspender vor dem Austrag zu schütteln ist. Untersuchungen im Bereich von Spendern mit pharmazeutischen Medien haben jedoch gezeigt, dass diese Anweisung von einem relevanten Teil der Benutzer nicht ausreichend ernst genommen wird, so dass der Austrag ohne vorheriges Schütteln des Mediumspenders oder nach nicht ausreichendem Schütteln des Spenders erfolgt. Aus US 5 363 842 A ist dem Fachmann bekannt, batteriebetriebene Schüttelerkennungsmechanismen mit entsprechenden Sensoren, die einen Schüttelvorgang erkennen können, zu versehen.

### Aufgabe und Lösung

Aufgabe der Erfindung ist es daher, einen Mediumspender gattungsgemäßer Art zur Verfügung zu stellen, der die Wahrscheinlichkeit erhöht, dass der Benutzer das Medium im gewünschten Mischungsverhältnis der Teilkomponenten des Mediums austrägt. Die Aufgabe wird durch einen Mediumspender mit den Merkmalen des Anspruch 1 gelöst. Erfindungsgemäß wird dies dadurch erreicht, dass der Mediumspender eine Erfassungseinrichtung zur Erfassung einer Schüttelbewegung aufweist, die eine haptische, optische oder akustische Ausgabeeinrichtung umfasst und die derart ausgebildet ist, dass sie in Reaktion auf ein manuelles Schütteln des Spenders die Ausgabeeinrichtung von einem ersten Zustand in einen zweiten Zustand überführt.

Ein erfindungsgemäßer Mediumspender informiert den Benutzer darüber, ob der Spender in ausreichendem Maße geschüttelt wurde. Dies kann in optischer Weise, beispielsweise über eine in Reaktion auf das Schütteln aktivierte oder deaktivierte LED, oder auch in akustischer Weise, beispielsweise über einen Signaltongeber, erfolgen. Es hat sich gezeigt, dass Spender, die Auskunft über den getätigten Schüttelvorgang geben, üblicherweise nicht im ungeschüttelten oder unzureichend geschüttelten Zustand verwendet werden. Während die häufig recht vagen Angaben in der Handhabungsanweisung zu Spendern, die sich meist auf die Zahl der Schüttelvorgänge oder den Schüttelzeitraum beziehen, aufgrund ihrer Unbestimmtheit dem Benutzer keinen klaren Hinweis darauf geben, in welchem Maße tatsächlich geschüttelt werden muss, führt ein für den Benutzer klar identifizierbares Signal, das den Bereitschaftszustand verdeutlicht, zu einem ausreichenden Schütteln und somit zum bestimmungsgemäßen Austrag des Mediums.

Die Erfassungseinrichtung eines erfindungsgemäßen Spenders ist derart ausgebildet, dass sie in der gewünschten Weise auf einen Schüttelvorgang reagiert. Dabei ist eine Vielzahl von Varianten denkbar.

So ist eine besonders einfache Gestaltung derart ausgebildet, dass die Erfassungseinrichtung einen vollständig gegenüber der Umgebung isolierten Indikatorraum mit einer zumindest teilweise transparenten Wandung aufweist, wobei dieser eine optische Ausgabeeinrichtung bildet und für einen Benutzer zumindest partiell einsehbar ist, wobei innerhalb des Indikatorraums ein Indikatormedium angeordnet ist, das durch das Schütteln sein Aussehen verändert.

Bei einer solchen Gestaltung ist somit ein vom Mediumspeicher vollständig isolierter Indikatorraum vorgesehen, der ein nicht für den Austrag bestimmtes Indikatormedium enthält. Dieses ändert durch Schütteln sein Aussehen. Der Benutzer kann durch die transparente Wandung hindurch in den Indikatorraum hineinschauen und somit den Zustand des Indikatormediums erkennen. Das Indikatormedium ist dabei derart ausgebildet, dass es nach Beendigung des Schüttelvorgangs wieder sein ursprüngliches Aussehen einnimmt, wobei dies vorzugsweise und bedingt durch die Art des Indikatormediums langsam und über einen Zeitraum von vorzugsweise einigen Minuten zu seinem ursprünglichen Aussehen zurückkehrt.

Damit das Indikatormedium durch Schütteln sein Aussehen verändert, sieht eine besonders vorteilhafte Gestaltung vor, dass es sich bei dem Indikatormedium um eine Emulsion oder Suspension handelt, also um ein aus einer Trägerflüssigkeit und einer zweiten Flüssigkeit oder Schwebstoffen bestehenden Medium. In der Art, wie man es von Souvenir-Glaskugeln kennt, in denen durch Schütteln Schneefall bewirkt werden kann, können somit durch Schütteln des Spenders Schwebstoffe oder Bläschen der zweite Flüssigkeit aufgewirbelt werden, so dass sie die Anmutung des Indikatormediums zumindest in jenem Bereich verändert, der für den Benutzer einsehbar ist. So könnte beispielsweise eine farblose Trägerflüssigkeit vorgesehen sein, in der eingefärbte Schwebstoffe enthalten sind. Im Ruhezustand des Spenders setzen sich diese Schwebstoffe am vorzugsweise für den Benutzer nicht einsehbaren Boden des Indikatorraums ab. Durch Schütteln des Spenders werden sie jedoch aufgewirbelt, so dass sie in den einsehbaren Bereich gelangen. Neben dem Sichtfenster, durch das der Benutzer in den Indikatorraum hineinschauen kann, kann zusätzlich eine Vergleichsfarbfläche vorgesehen sein. Sobald der Spender in ausreichendem Maße geschüttelt wurde, weist das durch das Sichtfenster hindurch erkennbare Indikatormedium die Farbe der Vergleichsfarbfläche auf.

Bei dieser Gestaltung mit Indikatormedium ist das Aussehen der Flüssigkeit im Flüssigkeitsspeicher der Indikator für eine ausreichende Durchmengung im Mediumspeicher des Mediumspenders. Eine Anpassung an verschiedene im Mediumspeicher verwendbare Medien kann dadurch erfolgen, dass die Flüssigkeiten im Flüssigkeitsspeicher hinsichtlich ihrer Viskosität oder ihrer Dichte angepasst werden oder indem die Dichte oder die Menge der Schwebstoffe angepasst wird. Hierdurch ist die Sinkgeschwindigkeit der Schwebstoffe beeinflussbar, die wiederum den Zeitraum beeinflusst, über den nach einem Schüttelvorgang die Schwebstoffe für den Benutzer durch das Sichtfenster hindurch sichtbar sind.

Bei einer anderen Variante eines erfindungsgemäßen Mediumspenders ist vorgesehen, dass die Ausgabeeinrichtung als optische Ausgabeeinrichtung ausgebildet ist und ein mechanisch gegenüber dem Gehäuse bewegliches Anzeigeelement aufweist, wobei die Position des Anzeigeelements durch Schütteln der Erfassungseinrichtung veränderbar ist und von außen für einen Benutzer erkennbar ist.

Bei einem solchen Spender ist somit ein gegenüber dem Gehäuse verlagerbarer Abschnitt in Form des Anzeigeelements vorgesehen, dessen Position für den Benutzer erfassbar ist. Diese Erfassbarkeit kann insbesondere dadurch unterstützt werden, dass am Anzeigeelement eine farblich hervorgehobene Fläche vorhanden ist, die in Abhängigkeit der Position des Anzeigeelements von außen sichtbar bzw. nicht sichtbar ist. Vorzugsweise sind zwei solche Farbflächen vorgesehen, wobei eine erste, vorzugsweise rote einen nicht ausreichend geschüttelten Zustand symbolisiert und eine zweite, vorzugsweise grüne einen ausreichend geschüttelten Zustand symbolisiert. Im ungeschüttelten Zustand ist dann nur oder vorwiegend die erste Farbfläche sichtbar, während im ausreichend geschüttelten Zustand nur oder vorwiegend die zweite Farbfläche sichtbar ist.

Die Position des Anzeigeelements kann durch Schütteln verändert werden. Hierbei kann insbesondere eine Verlagerung des Anzeigeelements genutzt werden, die sich beim Schütteln des Spenders aufgrund der Massenträgheit des Anzeigeelements ergibt.

Vorzugsweise ist das Anzeigeelement zwischen einer ersten Position, die einen ersten Schüttelzustand symbolisiert, und einer zweiten Position, die einen zweiten Schüttelzustand symbolisiert, beweglich. Dabei ist das Anzeigeelement durch Schütteln nur in Richtung der zweiten Position verlagerbar. Bei einer solchen Gestaltung ist somit vorgesehen, dass das Anzeigeelement zwischen einer den nicht oder nicht ausreichend geschüttelten Zustand symbolisierenden ersten Position und einer den ausreichend geschüttelten Zustand symbolisierten zweiten Position verlagerbar ist, wobei durch das Schütteln des Mediumspenders lediglich eine Verlagerung in Richtung der zweiten Position erfolgt. Diese durch das Schütteln bewirkte unidirektionale Bewegung des Anzeigeelements lässt sich insbesondere durch Rastelemente bzw. eine Rastleiter erreichen, die insbesondere durch eine sägezahnähnliche Gestaltung die Unidirektionalität gewährleisten kann. Um das Anzeigeelement bis in die zweite Position zu verlagern, muss es schrittweise über eine Mehrzahl von Schüttelperioden in Richtung der zweiten Position bewegt werden, wobei eine Rückverlagerung in Richtung der ersten Position zunächst nicht erfolgt bzw. durch Schütteln alleine nicht verursacht werden kann.

Um allerdings zu gewährleisten, dass das Anzeigeelement nicht für einen längeren Zeitraum in der zweiten Position verharrt und somit nach Ablauf einer gewissen Zeitspanne fälschlicherweise anzeigt, dass eine Austragbereitschaft besteht, sind vorzugsweise Rückstellmittel vorgesehen, die bewirken, dass das Anzeigeelement von einer der ersten Position abweichenden Position in die erste Position zurück überführt wird, sobald ein Haltezeitraum abgelaufen ist. Bei einem solchen Haltezeitraum kann es sich vorzugsweise um einen Zeitraum von einigen Sekunden, beispielsweise zwischen 10 Sekunden und 240 Sekunden handeln. Wenn nach dem Schütteln des Spenders der Spender somit ohne Bewirkung eines Austragvorgangs stehen bleibt, kehrt das Anzeigeelement nach Ablauf dieses Haltezeitraums in seine erste Position zurück. Es sind sowohl Gestaltungen denkbar bei denen lediglich aus der zweiten Position eine Rückbewegung in die erste Position nach Ablauf des Haltezeitraums bewirkt wird, als auch solche, bei denen jede von der ersten Position abweichende Position des Anzeigeelements nach Ablauf eines Haltezeitraums zur genannten Rückstellung führt.

Eine konstruktive Möglichkeit, dieses Merkmal zu realisieren, liegt darin, die die Unidirektionalität gewährleistende Rastleiter mit einem oder mehreren leicht schräg gestellten Rastzähnen zu versehen, entlang derer ein mit der Rastleiter im Eingriff befindlicher Eingriffabschnitt über den Haltezeitraum hinweg abgleitet, um nach Ablauf des Haltezeitraums vollständig den Berührkontakt zum jeweiligen schräg gestellten Rastzahn zu verlieren.

Weiterhin kann auch eine Wirkkopplung zwischen der Betätigungshandhabe und dem Anzeigeelement vorgesehen sein, durch die das Anzeigeelement zurück in die erste Position verlagert wird, wenn die Betätigungshandhabe betätigt wird. Somit wird nach Durchführung eines Austragvorgangs automatisch wieder ein Zustand des Anzeigeelements hergestellt, der den Schüttelbedarf symbolisiert.

Neben den genannten rein mechanischen Lösungen sind auch elektrisch unterstützte Lösungen eines erfindungsgemäßen Mediumspenders denkbar.

So weist die Erfassungseinrichtung des Mediumspenders bei solchen Lösungen vorzugsweise eine Ausgabeeinrichtung auf, die als elektrisch aktivierbare Ausgabeeinrichtung ausgebildet ist. Im Falle einer akustischen Ausgabeeinrichtung kann diese somit einen Lautsprecher enthalten. Bei einer optischen Ausgabeeinrichtung sind vorzugsweise eine oder mehrere LEDs vorgesehen, die den aktuellen Zustand des Spenders darstellen. Eine haptische Ausgabeeinrichtung könnte beispielsweise in Form eines Vibrators vorgesehen sein, der derart ausgestaltet ist, wie es von Mobiltelefonen bekannt ist.

Im Falle der Verwendung von LEDs oder einer anderweitigen optischen Ausgabeeinrichtung ist es insbesondere von Vorteil, wenn die Ausgabeeinrichtung zur Darstellung von mindestens drei für den Benutzer differenzierbaren Zuständen ausgebildet ist. Dies kann beispielsweise über eine LED erfolgen, die neben dem ausgeschalteten Zustand auch in zwei unterschiedlichen Farben Licht abgeben kann. Auch eine Gestaltung mit zwei LEDs ist in der Lage, die gewünschten drei differenzierbaren Zustände darzustellen. Die Möglichkeit, über die Ausgabeeinrichtung drei differenzierbare Zustände darzustellen, gestattet es, neben einem Ruhezustand auch einen weiteren Zustand für den Benutzer erfassbar zu machen, in welchem der Beginn des Schüttelvorgangs registriert wurde, der erfolgte Schüttelvorgang jedoch noch nicht ausreicht. Bei der Gestaltung mit einer LED könnte diese beispielsweise mit Beginn des Schüttelvorgangs eine erste Farbe annehmen und nach ausreichendem Schütteln eine zweite Farbe annehmen.

Zur Bestromung der elektrischen Ausgabeeinrichtung kann der Mediumspender mit einem Energiespeicher wie einer Batterie oder einem Akkumulator ausgestattet sein, der vorzugsweise im Lieferzustand bereits ausreichend befüllt ist, um über den Verwendungszeitraum des Spenders ausreichende Energie bereitzustellen. Nachteilig hieran ist jedoch, dass bei gattungsgemäßen Spendern der Zeitraum zwischen der Herstellung und der Auslieferung an den Benutzer und/oder der Zeitraum, innerhalb dessen der Spender durch den Benutzer verwendet wird, viele Monate betragen kann, so dass ein einmalig geladener Akkumulator oder eine einmalig geladene Batterie möglicherweise entleert ist, bevor der Mediumspeicher des Mediums vollständig entleert wurde.

Eine vorteilhafte Gestaltung des Mediumspenders sieht daher vor, dass die Erfassungseinrichtung einen Energiespeicher zur Speicherung elektrischer Energie und einen Energiewandler umfasst, der durch Schütteln eingebrachte mechanische Energie in elektrische Energie wandelt, wobei die so erzeugte elektrische Energie im Energiespeicher gespeichert wird. Bei einer solchen Gestaltung ist somit vorgesehen, dass die durch das Schütteln in das System eingebrachte Energie selbst verwendet wird, um einen Energiespeicher zu laden. Beim Energiespeicher kann es sich um den bereits genannten Akkumulator handeln. Es können jedoch auch Energiespeicher Verwendung finden, die lediglich eine kurzfristige Speicherung von Energie gestatten, wie beispielsweise ein Kondensator. Der Energiewandler kann in verschiedener Art ausgebildet sein. So ist beispielsweise die von "Schüttel-Taschenlampen" bekannte Gestaltung denkbar, bei der ein Magnet relativ zu einer Spule verlagert wird, so dass in die Spule eine Spannung induziert wird. Die Bewegung des Magneten oder der Spule kann dabei beispielsweise linear oder rotatorisch erfolgen. Auch eine auf Piezotechnik basierende Erzeugung elektrischer Energie ist denkbar, beispielsweise mittels eines piezoelektrischen Biegewandlers.

Die Umwandlung der mechanisch in das System eingebrachten Energie in elektrische Energie gestattet eine besonders einfache Gestaltung eines erfindungsgemäßen Mediumspenders. Bei dieser Gestaltung ist die Ausgabeeinrichtung mit dem genannten Energiespeicher über eine Schaltung verbunden ist, die eine Trennung des Energiespeichers von der Ausgabeeinrichtung bewirkt, solange die im Energiespeicher gespeicherte Energie ein bestimmtes Maß nicht überschreitet. Erst wenn ein Grenzwert, der beispielsweise ein Spannungs-Grenzwert oder ein Energie-Grenzwert sein kann, überschritten wurde, wird die Ausgabeeinrichtung bestromt. Bei einer solchen Gestaltung, bei der der Grenzwert-Schalter quasi einen Vergleicher bildet, kann somit gegebenenfalls vollständig auf einen Mikroprozessor verzichtet werden, da alleine die in die Energiespeicher durch das Schütteln eingebrachte Energie in Verbindung mit dem Grenzwert-Schalter ermöglicht, dass der Benutzer erkennt, ob er bereits in ausreichendem Maße geschüttelt hat.

Durch die Verwendung zweier Grenzwert-Schalter, die verschiedene Grenzwerte aufweisen und die Ausgabeeinrichtung in verschiedener Art beeinflussen, kann auch die oben beschriebene Gestaltung erreicht werden, bei der mit Beginn des Schüttelvorgangs eine erste LED erleuchtet wird und nach ausreichend langem Schüttelvorgang eine zweite LED erleuchtet wird, wobei diese zweite LED die Austragbereitschaft signalisiert.

Von besonderem Vorteil ist es, wenn bei dieser Gestaltung die Betätigungshandhabe derart mit dem Energiespeicher wirkgekoppelt ist, dass dieser entladen wird, sobald die Betätigungshandhabe betätigt wird. So kann die Betätigungshandhabe beispielsweise mittelbar oder unmittelbar einen als Energiespeicher verwendeten Kondensator kurzschließen. Somit ist nach dem Austragvorgang der Energiespeicher wieder leer, so dass es eines neuen Schüttelvorgangs bedarf, um die Bereitschafts-LED wieder zu aktivieren.

Neben einer solchen vergleichsweise einfachen Gestaltung ist jedoch auch eine Gestaltung eines erfindungsgemäßen Mediumspenders möglich, bei der die Erfassungseinrichtung einen Mikroprozessor umfasst, der zur Steuerung der Ausgabeeinrichtung mit dieser verbunden ist. Ein solcher Mikroprozessor kann durch einen bereits im Lieferzustand geladenen Energiespeicher mit elektrischer Energie versorgen. Er kann jedoch auch durch einen Energiespeicher mit elektrischer Energie versorgt werden, der durch den Schüttelvorgang des Spenders befüllt wird.

Der Mikroprozessor kann ähnlich des oben genannten Grenzwert-Schalters beim Vorhandensein eines Energiespeichers, der durch Schütteln geladen wird, dessen Füllstand zum Kriterium für die Aktivierung der Ausgabeeinrichtung machen. Durch die Verwendung eines Mikroprozessors ist jedoch auch eine Gestaltung denkbar, bei der ein selbst keine nennenswerte Energiemenge liefernder Beschleunigungssensor Verwendung findet, dessen Sensordaten vom Mikroprozessor ausgewertet werden, um zu erfassen, ob der Spender in ausreichendem Maß geschüttelt wurde. Hierbei könnte im einfachsten Falle der Zeitraum ausgewertet werden, innerhalb dessen geschüttelt wurde. Auch die Anzahl der Schüttelperioden könnte ausgewertet werden. Am vorteilhaftesten ist es jedoch, wenn die eingebrachte Energie mittels des Beschleunigungssensors erfasst und aufsummiert wird, bis ein gewünschter Grenzwert erreicht wurde.

Auch bei einer solchen Gestaltung mit einem Mikroprozessor kann eine Wirkkopplung mit der Betätigungshandhabe vorgesehen sein, durch die nach einem Betätigungsvorgang oder einer definierten Zahl von Betätigungsvorgängen ein Zurücksetzen des Mikroprozessors dahingehend erfolgt, dass dieser für einen erneuten Austragvorgang zunächst wieder vom ungeschüttelten Zustand des Spenders ausgeht.

Die Erfassungseinrichtung kann in das Gehäuse des Mediumspenders, welches vorzugsweise auch den Mediumspeicher beinhaltet, unmittelbar integriert sein. Dabei kann eine gegebenenfalls im Spender vorhandene Elektronik die beschriebenen Funktionen mit übernehmen. Bei Spendern, der elektronisch eine Verhinderung eines Austragvorgangs ermöglichen, beispielsweise um eine Überdosierung zu verhindern, kann auch vorgesehen sein, dass diese Verhinderung solange gegeben ist, solange der Spender noch nicht in ausreichendem Maße geschüttelt wurde. Dies bietet sich insbesondere bei Spendern an, bei denen der Fördervorgang elektrisch gespeist wird, beispielsweise bei solchen, die mittels einer Vibrationseinrichtung das Medium zerstäuben und dabei austragen. Bei diesen kann die Speisung der Fördereinrichtung oder der Vibrationseinrichtung mit elektrischer Energie davon abhängig gemacht werden, ob der Spender zuvor in ausreichendem Maße geschüttelt wurde.

Von besonderem Vorteil ist es, wenn die Erfassungseinrichtung als separates Modul ausgebildet ist, insbesondere als separates Modul mit einem von einem Hauptgehäuse des Mediumspenders getrennten Modulgehäuse. Die separate Gestaltung der Erfassungseinrichtung als Modul gestattet es, diese in unveränderter oder weitgehend unveränderter Form in verschiedenen Spendern zu verwenden. Unter dieser modularen Gestaltung ist zu verstehen, dass alle für die Funktionsweise der Erfassungseinrichtung erforderlichen Komponenten, also insbesondere die Ausgabeeinrichtung und jener Mechanismus, der beim Schütteln eine Zustandsänderung der Ausgabeeinrichtung bewirkt, in einer Baueinheit umfasst sind, die als ganzes gehandhabt werden kann. Wenn diese ein separates Modulgehäuse umfasst, so ist die Anbringung auch an der Außenseite eines Spenders möglich.

Somit kann die identische Erfassungseinrichtung oder zumindest Erfassungseinrichtungen, die lediglich geringfügig modifiziert sind, an verschiedenen Spendern Verwendung finden. Auch ist es so möglich, dass der Benutzer eine Erfassungseinrichtung nacheinander mit mehreren Mediumspendern verwendet.

Die Kopplungseinrichtung, mittels dessen das separate Modul mit dem Hauptgehäuse verbindbar ist, kann abhängig davon, wer bestimmungsgemäß den Wechsel durchführt, verschieden ausgebildet sein. Im Falle einer Erfassungseinrichtung, die für mehrere Spendertypen vorgesehen ist, jedoch durch den Endanwender nicht austauschbar sein soll, kann die Kopplungseinrichtung von außen unzugänglich angeordnet sein. Im Falle eines bestimmungsgemäßen Austausches durch den Benutzer ist die Kopplungseinrichtung vorzugsweise für den Benutzer zugänglich und leicht handhabbar.

### Kurzbeschreibung der Zeichnungen

Weitere Vorteile und Aspekte der Erfindung ergeben sich außer aus den Ansprüchen aus der nachfolgenden Beschreibung bevorzugter Ausführungsbeispiele der Erfindung, welche anhand der Figuren erläutert sind. Dabei zeigen:
- Fig. 1: einen erfindungsgemäßen Mediumspender mit einer erfindungsgemäßen Erfassungseinrichtung,
- Fig. 2: eine erste Ausgestaltung der Erfassungseinrichtung des Mediumspenders der Fig. 1,
- Fig. 3: eine zweite Ausführungsform der Erfassungseinrichtung des Mediumspender der Fig. 1,
- Fig. 3a, 3b: eine alternative Schaltung zur Verwendung in der Erfassungseinrichtung des Ausführungsbeispiels der Fig. 3,
- Fig. 4: eine dritte Ausführungsform der Erfassungseinrichtung des Mediumspeichers der Fig. 1 und
- Fig. 5a - 5c und Fig. 6: eine vierte Ausgestaltung der Erfassungseinrichtung des Mediumspenders der Fig. 1.

### Detaillierte Beschreibung der Ausführungsbeispiele

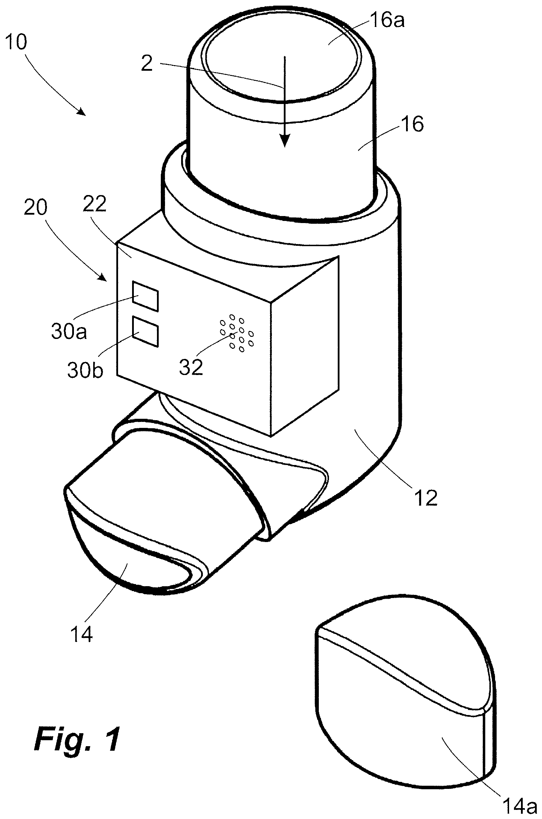

Fig. 1 zeigt einen erfindungsgemäßen Mediumspender 10. Dieser ist vorliegend als "Metered Dose Inhaler" (MDI) ausgebildet, wobei dieser Ausgestaltung vorliegend lediglich stellvertretend für eine Vielzahl möglicher Formgebungen eines erfindungsgemäßen Mediumspenders zu verstehen ist.

Der Mediumspender 10 verfügt über ein Gehäuse 12 mit einer durch eine Kappe 14a abdeckbaren Austragöffnung 14 zur oralen Verabreichung eines Medikaments. Für dieses Medikament ist ein Mediumspeicher 16 vorgesehen, der in das Gehäuse 12 eingeschoben ist und als ganzes in einer Betätigungsrichtung 2 niedergedrückt werden kann, um einen Austragvorgang zu bewirken. Die obere Abschlussfläche 16a des Mediumspeichers 16 bildet somit die Betätigungshandhabe 16a zur Auslösung eines Austragvorgangs. Im Falle des dargestellten Spenders 10 wird dieser Austragvorgang nach Öffnen eines Auslassventils durch die Betätigungsbewegung durch ein Treibmittel bewirkt. Die erfindungsgemäße Besonderheit, die nachfolgend erläutert wird, kann jedoch ebenfalls bei Spendern Verwendung finden, bei denen die zum Austrag erforderliche Energie beispielsweise unmittelbar über die mechanische Betätigung eingebracht wird oder aus einen elektrischen Energiespeicher stammt.

Innerhalb des Mediumspeichers 16 ist ein auszutragendes Medikament in Form einer Suspension enthalten. Es ist gewünscht, dass vor einen Austragvorgang eine Durchmengung dieser Suspension durch Schütteln des Spenders 10 bewirkt wird.

An der Vorderseite des Mediumspenders 10 ist eine Erfassungseinrichtung 20 vorgesehen, die der Erfassung einer Schüttelbewegung dient. Diese Erfassungseinrichtung 20 verfügt bei der beispielhaften Ausgestaltung, die in Fig. 1 gezeigt ist, über ein eigenes Modulgehäuse 22 und über zwei LEDs 30a, 30b sowie einen Lautsprecher 34.

Sobald der Spender 10 durch einen Benutzer geschüttelt wird, wird die LED 30a bestromt, so dass diese leuchtet und dem Benutzer dadurch anzeigt, dass der Schüttelvorgang registriert wurde. Sobald der Spender 10 in ausreichendem Maße geschüttelt wurde, um eine gewünschte Durchmengung des pharmazeutischen Mediums im Mediumspeicher 16 zu bewirken, beispielsweise für einen Zeitraum von etwa 20 Sekunden bei einer Frequenz von etwa 2 Hz, wird auch die zweite LED 30b bestromt und es wird ein Signalton über den Lautsprecher 32 ausgegeben, so dass der Benutzer weiß, dass das erfolgte Schütteln ausreicht. Der Benutzer kann nun in der bestimmungsgemäßen Weise die Austragöffnung 14 an seinem Mund platzieren und mittels der Betätigungshandhabe 16a ein Austragvorgang des Mediums aus dem Flüssigkeitsspeicher 16 bewirken.

Die Erfassungseinrichtung 20 kann als separates Modul ausgebildet sein, welches im vollständig montierten Zustand an das Spendergehäuse 12 angekoppelt werden kann. Hierzu können nicht näher dargestellte mechanische Kopplungsschnittstellen am Hauptgehäuse 12 und dem Modulgehäuse 22 vorgesehen sein. Weiterhin kann vorgesehen sein, dass zur Registrierung eines Austragvorgangs Durchbrechungen im Modulgehäuse 22 und dem Hauptgehäuse 12 vorgesehen sind, durch die hindurch der Erfassungseinrichtung die Bewegung der Betätigungshandhabe 16a mechanisch oder elektrisch erfasst.

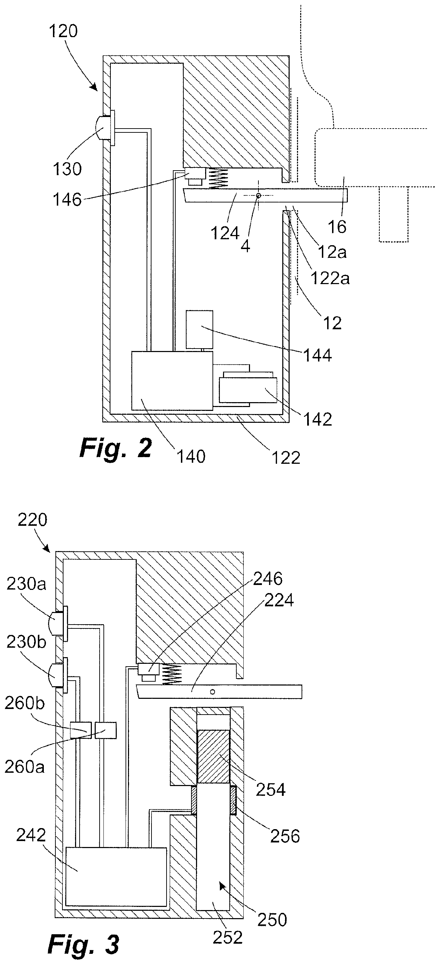

Fig. 2 zeigt eine erste Variante 120 einer Erfassungseinrichtung 20. Diese Erfassungseinrichtung 120 verfügt über ein Gehäuse 122, welches weitgehend geschlossen ist und die externe Anbringung an einem weitgehend unveränderten üblichen Spender gestattet. Innerhalb des Gehäuses 122 ist eine Mikroprozessor 140 vorgesehen, der von einer Batterie 142 mit elektrischer Energie versorgt wird. Dieser Mikroprozessor 140 ist darüber hinaus mit einem Beschleunigungssensor 144 sowie einer Anzeige-LED 130 verbunden.

Wenn der Spender 10 vom Benutzer geschüttelt wird, so wird dies vom Sensor 144 erfasst und es werden entsprechende Sensorausgangsdaten an den Mikroprozessor 140 weitergegeben. Dieser wertet die erfassten Daten aus, beispielsweise im Hinblick auf die Anzahl der Schüttelperioden und/oder im Hinblick auf die Stärke des Schüttelns. Sobald der Schüttelvorgang ein Maß erreicht hat, welches eine ausreichende Durchmischung der Flüssigkeit im Mediumspeicher 16 gewährleistet, wird die LED 130 bestromt, so dass der Benutzer hierdurch erfährt, dass der Spender 10 nun für einen Austragvorgang fertig vorbereitet ist.

Mit dem Mikroprozessor 140 ist weiterhin noch ein Taster 146 verbunden, der durch einen Schwenkhebel 124 niedergedrückt werden kann. Der Schwenkhebel ist um eine gehäusefeste Schwenkachse 4 schwenkbeweglich. Sein vom Taster 146 weg weisendes Ende ragt durch eine Aussparung 122a des Gehäuses 122 sowie durch eine Aussparung 12a des Gehäuse 12 hindurch, so dass beim er beim Niederdrücken des Mediumspeicher 16 im Uhrzeigersinn verschwenkt wird. Der Taster 146 gestattet es, den erfolgen Austragvorgang zum Rücksetzen des Mikroprozessors 140 zu verwendet. Für einen darauffolgenden nächsten Austragvorgang muss der Spender 10 somit erneut geschüttelt werden, bis die Austragbereitschaft durch die LED 130 angezeigt wird.

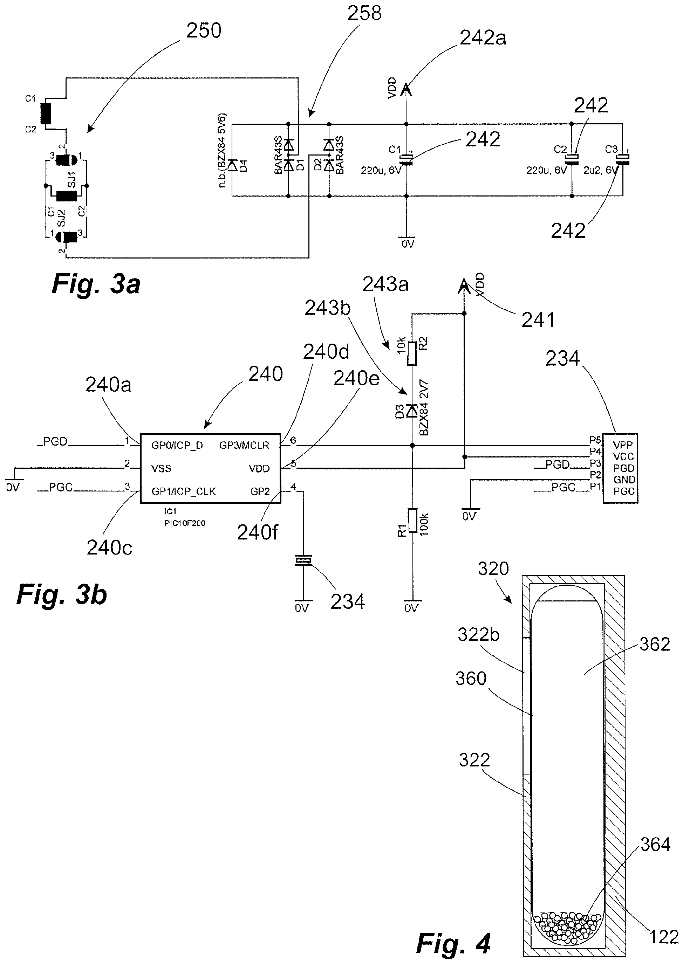

Fig. 3 zeigt eine weitere Variante 220 einer erfindungsgemäßen Erfassungseinrichtung 20. Diese Erfassungseinrichtung 220 weist wiederum zwei LEDs 230a, 230b auf, die in der zu Fig. 1 erläuterten Art und Weise der Informierung des Benutzers dienen. Weiterhin umfasst die Erfassungseinrichtung 220 einen Energiespeicher 242, welcher jedoch abweichend von der Batterie 142 der Ausführungsform der Fig. 2 im Lieferzustand des Spenders nicht geladen ist. Er ist stattdessen mit einem Energiewandler 250 verbunden. Dieser verfügt über einen vertikal erstreckten Schacht 252, in dem ein Magnet 254 beweglich gelagert ist. Dieser Schacht 252 wird von einer Spule 256 umgeben, in die Strom induziert wird, wenn sich der Magnet 254 im Schacht 252 bewegt. Die Bewegung des Magneten 254 wird durch Schütteln des Spenders 10 verursacht, so dass dieses Schütteln ein Laden des beispielsweise durch Kondensatorplatten gebildeten Energiespeichers 242 bewirkt.

Sobald die im Energiespeicher 242 vorhandene Energie einen vorgegebenen Grenzwert übersteigt, führt dies zu einem Schließen eines Grenzwert-Schalters 260a, so dass ab diesem Zeitpunkt die LED 30a bestromt wird. Der entsprechende Grenzwert ist sehr niedrig gesetzt, so dass im Idealfall bereits nach ein bis zwei Schüttelbewegungen die LED 30a aufleuchtet. Der Benutzer bekommt hierdurch mitgeteilt, dass der Beginn des Schüttelvorgangs registriert wurde. Der zweite Grenzwert-Schalter 260b, der den Energiespeicher 242 mit LED 30b verbindet, schließt bei einem deutliche höheren Grenzwert. Erst wenn die Energie im Energiespeicher 242 ein Niveau erreicht hat, welches sich beispielsweise durch mittelstarkes bis starkes Schütteln des Spenders für 20 Sekunden einstellt, schließt der Schalter 260b, so dass die LED 30b dann aktiviert wird. Dies signalisiert dem Benutzer, dass er nun mit dem Austragvorgang beginnen kann, indem er die Betätigungshandhabe 16a hinunterdrückt.

In ähnlicher Weise wie die Erfassungseinrichtung der Fig. 2 weist auch die Erfassungseinrichtung der Fig. 3 einen zusätzlichen Taster 246 auf, der über einen Hebel 224 eine Wirkkopplung mit dem Mediumspeicher 16 bildet. Sobald der Mediumspeicher 16 hinabgedrückt wird, schließt der Taster 246 den Energiespeicher 242 kurz, beispielsweise durch Verbinden zweier Kondensatorplatten des Energiespeichers 242. Somit wird durch Betätigen des Spenders 10 wieder ein Ausgangszustand erzielt, in dem der Energiespeicher 242 entladen ist und von dem ausgehend es zunächst eines erneuten Schüttelns bedarf, um den Spender 10 in einen austragfertigen Zustand zu versetzen.

Fig. 3a und 3b zeigen eine elektronische Schaltung, mittels derer die zu Fig. 3 beschriebene Funktionalität realisiert sein kann.

Wie in Fig. 3a dargestellt ist, verfügt die Schaltung über einen mechanoelektrischen Energiewandler 250, der beim Schütteln des Spenders eine Wechselspannung erzeugt. Mit dem Energiewandler 250 ist ein Brückengleichrichter 258 verbunden, der die Wechselspannung in eine Gleichspannung überführt, welche zum Aufladen der als Energiespeicher agierenden Kondensatoren 242 genutzt wird. Die Kondensatoren 242 wirken als Energieintegrator. Durch fortgesetzte Schüttelbewegung kann die in den Kondensatoren gespeicherte elektrische Energie erhöht werden. Mit der gespeicherten Energie steigt auch die Ausgangsspannung der Kondensatoren 242 am Ausgangsanschluss 242a.

Der Ausgangsanschluss 242a der Kondensatoren 242 ist mit einem Eingangsanschluss 241 einer Auswerte- und Anzeigeschaltung verbunden, die in Fig. 3b dargestellt ist. Der Eingangsanschluss 241 ist zum Zwecke der Stromversorgung zum einem mit einem Display 230 verbunden, welches zur Darstellung der Worte "Shake" und "Ready" ausgebildet ist. Gleichermaßen könnten hier auch LEDs Verwendung finden. Der Eingangsanschluss 241 ist weiterhin zum Zwecke der Stromversorgung unmittelbar mit einem Versorgungsanschluss 240e eines Mikroprozessors 240 verbunden. Über einen Spannungsteiler 243a und eine Zehnerdiode 243b ist der Eingangsanschluss 241 darüber hinaus mittelbar auch mit einem Digitalanschluss 240d des Mikroprozessors verbunden.

Der Mikroprozessor 240 verfügt über drei Ausgänge 240a, 240c, 240f. Am Ausgang 240f ist ein Summer 234 angeschlossen. Die Ausgänge 240a, 240c sind mit dem Display 230 verbunden und gestatten es, auf diesem mittels des Anschlusses 240a das Wort "Shake" und mittels des Anschlusses 240c das Wort "Ready" erscheinen zu lassen.

Die Funktionsweise der Schaltung der Fig. 3a und 3b ist die folgende:
Durch Schütteln des Spenders erzeugt der Energiewandler 250 einen elektrischen Wechselstrom, der vom Brückengleichrichter 258 gleichgerichtet und dann den Kondensatoren 242 zugeführt wird. Mit Anstieg der in den Kondensatoren gespeicherten elektrischen Energie steigt auch die Spannung am Ausgangsanschluss 242a und dem Eingangsanschluss 241. Sobald diese Spannung ein zum Betrieb des Mikroprozessors 240 ausreichendes Niveau erreicht hat, wird dieser gestartet und überwacht ab diesem Zeitpunkt den Digitaleingang 240d. Gleichzeitig wird über den Ausgang 240a im Display 232 das Wort "Shake" eingeblendet. Wenn durch fortgesetztes Schütteln das Energieniveau in den Kondensatoren 242 und mit ihm das Spannungsniveau am Eingangsanschluss 241 weiter erhöht wird, erreicht auch die Spannung jenseits des Spannungsteilers 243a und der Zehnerdiode 243b ein Niveau, welches zur Erkennung der Spannung am Digitaleingang 240d des Prozessors 240 führt.

Dies wird vom Prozessor dahingehend verstanden, dass ausreichend stark geschüttelt wurde. Über die Ausgänge 240b und 240f kann dieser Zustand durch optische Anzeige des Wortes "Ready" auf dem Display 234 und durch Summen des Summers 234 für den Benutzer erkennbar gemacht werden. Dieser kann den Spender nun bestimmungsgemäß verwenden.

Fig. 4 zeigt eine weitere Gestaltung einer erfindungsgemäß an einem Spender 10 vorgesehenen Erfassungseinrichtung 320. Diese ist besonders einfach ausgestaltet. Sie weist einen transparenten Speicher 360 für ein Indikatormedium auf, der gegenüber einer Umgebung vollständig isoliert ist. In diesem Speicher 360 befindet sich das Indikatormedium, welches neben einer Flüssigkeit 362 auch eine Vielzahl von festen Schwebeteilchen 364 enthält. In einem oberen Bereich ist das Gehäuse 322 mit einem Fenster 322b versehen, durch welches ein oberer Bereich des Speichers 360 einsehbar ist.

Wenn der Spender 10 nun gemeinsam mit der Erfassungseinrichtung 320 geschüttelt wird, werden die Schwebeteilchen 364, deren Dichte geringfügig größer als die der Flüssigkeit 362 ist, hochgewirbelt, so dass sie sich weitgehend homogen in der Flüssigkeit 362 verteilen. Die hochgewirbelten Schwebeteilchen 364 sind durch die Fensteröffnung 322b für den Benutzer erkennbar, wobei sie hierzu insbesondere mit einer charakteristischen Farbgebung, beispielsweise einer grünen Farbgebung, versehen sind. Wenn der Inhalt des Speichers 360 vom Benutzer durch die Öffnung 322b als entsprechend eingefärbt wahrgenommen wird, weiß er, dass in ausreichendem Maße geschüttelt wurde. Er kann den Spender 10 dann bestimmungsgemäß verwenden.

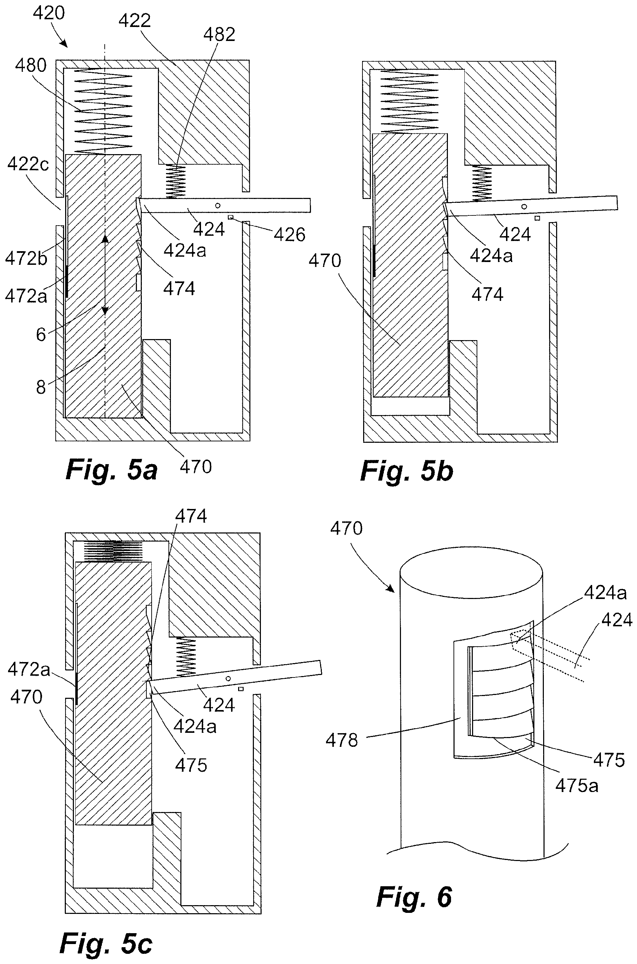

Die Ausgestaltung der Fig. 5a bis 5c und 6 stellt wiederum eine mechanische Lösung der Erfassungseinrichtung in stark schematisierter Form dar. Diese Erfassungseinrichtung 420 wird nachfolgend zunächst anhand der Fig. 5a erläutert. Die Veränderungen an der Erfassungseinrichtung 420, die durch das Schütteln des Spenders 10 bewirkt werden, werden anschließend anhand des Übergangs vom Zustand der Fig. 5a über den Zustand der Fig. 5b zum Zustand der Fig. 5c erläutert.

Innerhalb des Gehäuses 422 der Erfassungseinrichtung 420 ist ein Anzeigebolzen 470 mit etwa kreisförmigem Querschnitt positioniert. Dessen zur Vorderseite und einem dort angeordneten Fenster 422c weisende Seite weist eine grüne Farbfläche 472a und eine rote Farbfläche 472b auf. Wie anhand der Fig. 5a ersichtlich ist, ist im Ausgangszustand die rote Farbfläche 472b hinter dem Fenster 422c angeordnet, so dass der Benutzer erkennt, dass die Erfassungseinrichtung 420 sich im ungeschüttelten Ausgangszustand befindet. Das Anzeigeelement 470 ist in zweierlei Weise gegenüber dem Gehäuse 422 beweglich. Zum einen kann es in einer Längsrichtung 6 gegen die Kraft einer Feder 480 verschoben werden. Zum anderen kann es um eine Drehachse 8 verdreht werden, wobei es hierbei die Feder 480 tordiert. Auf seiner Rückseite ist das in Fig. 6 nochmals dargestellte Anzeigeelement 470 mit einer sägezahnartigen Rastleiter 474 versehen. In der aus Fig. 5 ersichtlichen Weise greift ein mit einer Schräge versehenes Ende 424a eines Schwenkhebels 424 in diese Rastleiter 474 ein. Der Schwenkhebel 424 ist dabei hinsichtlich seiner Schwenkbeweglichkeit durch einen Anschlag 426 begrenzt. Einer Schwenkverlagerung des Hebels 424 entgegen dem Uhrzeigersinn wirkt eine zweite Feder 482 entgegen.

Wenn ausgehend vom Zustand der Fig. 5a der Spender 10 geschüttelt wird, so wird dadurch in Verbindung mit der Massenträgheit des Anzeigeelements 470 eine Bewegung des Anzeigeelements 470 nach oben verursacht, wobei diese Bewegung gegen die Druckkraft der Feder 480 erfolgt. Wenn das Anzeigeelement dabei ausreichend weit bewegt wird, springt das Ende 424a des Schwenkhebels eine Raststufe der Rastleiter 474 weiter. Aufgrund der sägezahnartigen Gestaltung der Rastleiter 474 ist eine Rückkehr des Anzeigeelements 470 in seiner Ausgangslage der Fig. 5a danach zunächst nicht möglich. Der Zustand der Fig. 5b verdeutlicht dies. Wenn der Spender 10 mit der Erfassungseinrichtung 420 ausreichend lang geschüttelt wurde, stellt sich der Zustand der Fig. 5c ein. In diesem Zustand hat das Ende 424a des Schwenkhebels 424 die letzte Raststufe überwunden. Die grüne Farbfläche 472a ist daher jetzt hinter dem Fenster 422c positioniert, so dass dem Benutzer signalisiert wird, dass er in ausreichendem Maße geschüttelt hat.

Wenn der Benutzer nunmehr den Spender betätigt, so wird hierdurch ein Verschwenken des Hebels 424 im Uhrzeigersinn bewirkt, wobei dieser hierdurch aus dem Rastleiter 474 herausgehoben wird, so dass das Anzeigeelement 470 in die Ausgangslage der Fig. 5a zurückschnellen kann.

Um zu gewährleisten, dass der in Fig. 5c dargestellte Zustand, in dem dem Benutzer die Bereitschaft zum Austrag signalisiert wird, nicht für einen unbegrenzten Zeitraum erhalten bleibt, ist die letzte Sprosse 475 der Rastleiter 474 mit einer schrägen Fläche 475a versehen. Nach Erreichen des Zustandes der Fig. 5c und für den Fall, dass kein Austragvorgang bewirkt wird, gleitet das Ende 224a des Hebels 224 unter der Kraftwirkung der Federn 480, 482 daher langsam die Schräge 475a entlang, während das Anzeigeelement 470 sich langsam um die Achse 8 dreht und hierbei die Feder 480 tordiert. Hierdurch gelangt nach einer Zeitspanne, die durch verschiedene Parameter wie der Winkel der Schrägstellung, die Eigenschaften der Federn und die verwendete Reibpaarung, bedingt ist und die vorzugsweise zwischen 10 Sekunden und 240 Sekunden beträgt, das Ende 224a des Hebels 224 in eine verzahnungsfreie Rückstellnut 478. Sobald dies der Fall ist, schnellt das Anzeigeelement 470 zurück in seine Ausgangslage der Fig. 5a, obwohl es nicht zu einem Austragvorgang gekommen ist.

Um ausgehend von der Ausgangslage der Fig. 5a wieder einen austragfertigen Zustand zu bewirken, muss der Benutzer somit erneut den Mediumspender 10 schütteln.

## Patentansprüche

1. Transportabler Mediumspender (10) zum Austrag von flüssigen, pasteusen oder pulverförmigen Medien, mit
- einem Gehäuse (12) mit einer Austragöffnung (14),
- einem Mediumspeicher (16) zur Aufnahme des Mediums vor dem Austrag und
- einer Betätigungshandhabe (16a), mittels derer ein Fördervorgang bewirkt werden kann, bei dem Medium vom Mediumspeicher (16) zur Austragöffnung (14) gefördert wird,
wobei
der Spender (10) eine Erfassungseinrichtung (220) zur Erfassung einer Schüttelbewegung aufweist, die eine haptische, optische oder akustische Ausgabeeinrichtung (230a, 230b) umfasst und die derart ausgebildet ist, dass sie in Reaktion auf ein manuelles Schütteln des Spenders (10) die Ausgabeeinrichtung von einem ersten Zustand in einen zweiten Zustand überführt,
wobei
- die Erfassungseinrichtung einen Energiespeicher (242) zur Speicherung elektrischer Energie und einen Energiewandler (250) umfasst, der durch Schütteln eingebrachte mechanische Energie in elektrische Energie wandelt, wobei die so erzeugte elektrische Energie den Energiespeicher (242) lädt, und
- die Ausgabeeinrichtung (230a, 230b) mit dem Energiespeicher (242) über eine Schaltung (260a, 260b) verbunden ist, die eine Trennung des Energiespeichers (242) von der Ausgabeeinrichtung (230a, 230b) bewirkt, solange die im Energiespeicher gespeicherte Energie einen Grenzwert nicht überschreitet.

2. Mediumspender nach Anspruch 1,
**dadurch gekennzeichnet, dass**
die Ausgabeeinrichtung (130; 230a, 230b) als elektrisch aktivierbare Ausgabeeinrichtung ausgebildet ist.

3. Mediumspender nach Anspruch 2,
**dadurch gekennzeichnet, dass**
die Ausgabeeinrichtung (230a, 230b) als optische Ausgabeeinrichtung ausgebildet ist, wobei die Ausgabeeinrichtung vorzugsweise zur Darstellung mindestens drei differenzierbarer Zustände ausgebildet ist.

4. Mediumspender nach einem der Ansprüche 1 bis 3,
**dadurch gekennzeichnet, dass**
die Erfassungseinrichtung einen Mikroprozessor (140) umfasst, der zur Steuerung der Ausgabeeinrichtung (130) mit dieser verbunden ist.

## Claims

1. Portable medium dispenser (10) for discharging liquid, pasty or powdery media, comprising
- a housing (12) having a discharge opening (14),
- a medium store (16) for accommodating the medium before discharge, and
- an actuating handle (16a), by means of which a conveying process can be effected, in which medium is conveyed from the medium store (16) to the discharge opening (14),
wherein
the dispenser (10) has a detecting device (220) for detecting a shaking movement, said detecting device comprising a haptic, optical or acoustic output device (230a, 230b) and being designed in such a way that it transfers the output device from a first state into a second state in response to manual shaking of the dispenser (10),
wherein
- the detecting device comprises an energy store (242) for storing electrical energy and an energy converter (250), which, by shaking, converts introduced mechanical energy into electrical energy, wherein the electrical energy thus produced charges the energy store (242), and
- the output device (230a, 230b) is connected to the energy store (242) via a circuit (260a, 260b) which causes a separation of the energy store (242) from the output device (230a, 230b) as long as the energy stored in the energy store does not exceed a limit value.

2. Medium dispenser according to Claim 1,
**characterized in that**
the output device (130; 230a, 230b) is formed as an electrically activatable output device.

3. Medium dispenser according to Claim 2,
**characterized in that**
the output device (230a, 230b) is formed as an optical output device, wherein the output device is preferably designed to indicate at least three differentiable states.

4. Medium dispenser according to one of Claims 1 to 3,
**characterized in that**
the detecting device comprises a microprocessor (140), which is connected to the output device (130) for control thereof.

## Revendications

1. Distributeur de support pharmaceutique transportable (10) destiné à la distribution d'un support pharmaceutique liquide, pâteux ou pulvérulent, doté
- d'un boîtier (12) doté d'une ouverture de distribution (14),
- d'un stockage de support (16) destiné à accueillir le support avant distribution et
- d'une poignée (16a) au moyen de laquelle une opération de transport peut être effectuée, lors de laquelle le support est transporté du stockage de support (16) à l'ouverture de distribution (14),
dans lequel le distributeur (10) comporte un dispositif de détection (220) visant à détecter un mouvement d'oscillation et doté d'un dispositif d'émission haptique, optique ou acoustique (230a, 230b), le dispositif de détection étant conçu de façon à amener le dispositif d'émission d'un premier état à un second état en réaction à une oscillation manuelle du distributeur (10),
dans lequel
- le dispositif de détection comporte un accumulateur d'énergie (242) destiné à accumuler de l'énergie électrique et un convertisseur d'énergie (250), convertissant l'énergie mécanique apportée par les oscillations en énergie électrique, dans lequel l'énergie électrique ainsi obtenue charge l'accumulateur (242), et
- le dispositif d'émission (230a, 230b) est raccordé à l'accumulateur (242) par le biais d'un circuit (260a, 260b), effectuant une séparation de l'accumulateur (242) et du dispositif d'émission (230a, 230b), tant que l'énergie stockée dans l'accumulateur ne dépasse pas une valeur limite.

2. Distributeur de support pharmaceutique selon la revendication 1, **caractérisé en ce que** le dispositif d'émission (130 ; 230a, 230b) est conçu comme dispositif d'émission électriquement activable.

3. Distributeur de support pharmaceutique selon la revendication 2, **caractérisé en ce que** le dispositif d'émission (230a, 230b) est conçu comme dispositif d'émission optique, dans lequel le dispositif d'émission est conçu de préférence pour représenter au moins trois états pouvant être différenciés.

4. Distributeur de support pharmaceutique selon l'une des revendications 1 à 3, **caractérisé en ce que** le dispositif de détection comporte un microprocesseur (140), lequel est raccordé au dispositif d'émission (130) afin de le commander.
